# FASCICULE DE BREVET EUROPEEN

(11) **EP 0 774 990 B1**
(45) Date de publication et mention de la délivrance du brevet: **21.04.1999**
(21) Numéro de dépôt: 94919725.5
(22) Date de dépôt: 16.06.1994
(51) Int. Cl.: A61M 29/00, A61B 17/34, A61B 17/02

(54) **INSTRUMENT DE TRAITEMENT CHIRURGICAL D'UN DISQUE INTERVERTEBRAL PAR VOIE ANTERIEURE**
GERAET ZUR AEUSSEREN WIRBELSAEULENCHIRURGIE
INSTRUMENT FOR ANTERIOR INTERVERTEBRAL DISK SURGERY

(30) Priorité: 17.06.1993 FR 9307330
(43) Date de publication de la demande: 28.05.1997
(73) Titulaire: SOCIETE DE FABRICATION DE MATERIEL ORTHOPEDIQUE SOFAMOR, 75016 Paris (FR)
(72) Inventeur: BEURIER, Jacques, Pierre, F-76310 Sainte-Adresse (FR); CUNCI, Olivier, F-76600 Le Havre (FR)
(74) Mandataire: Martin, Jean-Paul
(86) Numéro de dépôt international: FR9400728
(87) Numéro de publication internationale: WO9500197

(56) Documents cités:
- EP-A- 0 138 089
- EP-A- 0 334 116
- WO-A-91/19528
- WO-A-92/21298
- DE-A- 3 936 811

## Description

La présente invention a pour objet un instrument de traitement chirurgical d'un disque intervertébral par voie antérieure, notamment par nucléotomie percutanee. Selon le préambule de la revendication 1, cet instrument est avantageusement utilisable dans le traitement par chirurgie coelioscopique du disque lombaire situé entre la cinquième vertèbre lombaire et le sacrum (communément appelé disque L5-S1).

Le traitement des disques intervertébraux s'est récemment développé grâce, entre autres, aux techniques de la chirurgie percutanée qui présente notamment l'avantage d'être "non invasive". En effet cette chirurgie provoque, aussi bien de la part des patients que des organismes hospitaliers ou de protection médicale, un très grand intérêt. Cet intérêt est principalement dû à l'obtention d'une amélioration très appréciable des suites opératoires et à une importante réduction du temps d'hospitalisation. En revanche, cette chirurgie est relativement onéreuse en matériel, et implique une spécialisation chirurgicale plus poussée, tant dans la salle d'opération que des chirurgiens spécialement formés.

Le document WO-A-92 21 298 décrit un instrument pour le traitement par voie interne d'une pluralité de structures du corps, par exemple un procédé laparoscopique, comportant un tube et une poignée de préhension manuelle.

Le principe de la nucléotomie percutanée consiste en une ponction du disque permettant de diminuer la pression intra-discale et éventuellement de retirer le contenu discal dégénéré, sans avoir à effectuer un véritable abord chirurgical. Jusqu'à présent seul l'abord postéro-latéral sous contrôle radioscopique a été utilisé, le patient étant placé en position de décubitus ventral ou éventuellement latéral. Le nucléus est ôté, soit en utilisant une pince (cas le plus fréquent), soit au moyen d'un laser (technique en développement actuellement) ou en utilisant un système motorisé permettant de fragmenter le disque et d'aspirer les débris.

Les indications sont essentiellement les compressions nerveuses par protrusions ou hernies discales intra-rachidiennes. Les sciatiques et les hernies discales non exclues sont indiquées à condition que le patient soit jeune et l'annulus postérieur sain.

Le plus souvent il s'agit des trois derniers disques lombaires : L3-L4 et surtout L4-L5 et L5-S1, la hernie de ces deux derniers disques étant responsable de la plupart des sciatiques discales "communes". Si de nombreux points techniques restent controversés (nécessité d'un discoscope, discectomie manuelle ou automatisée etc), un consensus à peu près général paraît acquis en ce qui concerne la technique d'abord percutanée des disques lombaires : jusqu'à présent, pour la totalité des auteurs, la ponction du disque doit être effectuée par voie postéro-latérale, sous contrôle radioscopique. La pénétration du dispositif de traitement se fait à l'angle postérolatéral du disque, en évitant le nerf rachidien, ce qui implique le recours à une anesthésie locale.

Les chirurgiens s'accordent généralement à reconnaître l'intérêt de cette technique opératoire, en raison de la relative facilité d'accès qu'elle confère, notamment pour les disques lombaires L3-L4 et L4-L5. En revanche, l'abord du disque L5-S1, disque qui fait actuellement l'objet de près de la moitié des opérations des disques lombaires, est plus difficile, en raison du caractère beaucoup moins superficiel de ce segment rachidien et de la nécessité de contourner l'aile iliaque. C'est pourquoi on a proposé divers artifices spécifiques pour l'abord du disque L5-S1 : instrument courbe, inclinaison rachidienne etc. Mais le taux de grandes difficultés techniques ou même d'échecs paraît néanmoins relativement élevé. Ces difficultés entraînent de plus une augmentation de la durée de l'intervention, de l'irradiation des patients ainsi que l'impossibilité d'utiliser des instruments de fort calibre.

L'invention a donc pour but de résoudre ce problème en proposant une solution à la fois simple et efficace.

Conformément à l'invention, l'instrument de traitement chirurgical comprend une aiguille-guide, au moins deux tubes gigognes concentriques rectilignes adaptés pour pouvoir être enfilés sur l'aiguille-guide et un tube extérieur de travail, concentrique aux tubes précités. Ces derniers peuvent coulisser dans ce tube extérieur qui est pourvu à son extrémité apicale, destinée à pénétrer dans le disque à traiter, d'un moyen d'écartement des vertèbres contiguës à ce disque et de maintien de cet écartement pendant le traitement chirurgical, tandis que son extrémité proximale est équipée d'une poignée de préhension manuelle.

Ainsi l'instrument selon l'invention est spécialement adapté pour l'abord du disque par voie antérieure et non plus par voie postéro-latérale. Cet instrument est essentiellement constitué d'un assemblage de tubes gigognes concentriques, dont le tube extérieur de travail est équipé d'un moyen d'écartement des vertèbres, ou de la cinquième vertèbre lombaire et du sacrum s'il s'agit du traitement du disque L5-S1. Grâce à cet écartement, on évite toute expulsion intempestive du tube du fait de l'hyperpression intersomatique. Le maintien de cet écartement pendant l'intervention permet d'exécuter aisément l'intervention sur le disque, notamment une nucléotomie, de manière efficace et rapide, sans nuire à l'environnement anatomique du patient.

Suivant un mode de réalisation de l'invention, le moyen d'écartement et de maintien des vertèbres écartées comprend une olivette faisant saillie du tube extérieur et fixée coaxialement à ce dernier à son extrémité apicale. Cette olivette présente deux plats latéraux symétriques, reliés à deux bossages arrondis symétriques, la distance entre les deux plats étant ainsi inférieure à la distance entre les sommets des bossages, afin que par rotation du tube extérieur sur 90° environ à partir de sa position dans laquelle les plats sont paralléles au plan général du disque à traiter, les corps vertébraux sous et sus jacents puissent être écartés et maintenus en position écartée.

Suivant un autre mode de réalisation possible de l'invention, le tube extérieur est double et constitué de deux éléments tubulaires concentriques pouvant coulisser axialement l'un par rapport à l'autre, la poignée de préhension est prévue sur l'extrémité proximale de l'élément tubulaire extérieur, dans l'extrémité apicale duquel est ménagée au moins une lumière, et l'extrémité apicale de l'élément intérieur est pourvue d'au moins une lame flexible élastiquement, faisant saillie extérieurement audit élément à l'état libre, escamotée lorsque l'élément intérieur est introduit à coulisse dans l'élément extérieur et faisant saillie de ce dernier à travers la lumière correspondante afin de constituer ledit moyen d'écartement des corps vertébraux, lorsque l'élément extérieur est dans la position angulaire voulue entre les corps vertébraux.

Dans les divers modes de réalisation possibles de l'invention, les moyens d'écartement sont tels qu'ils s'opposent le moins possible à la pénétration du tube dans le corps du malade, puis dans le disque à traiter. Ces moyens sont agencés pour pouvoir créer un écartement suffisant des vertèbres voisines et maintenir avec sécurité cet écartement lors de l'opération de nucléotomie.

Par rapport aux autres techniques chirurgicales que sont la discectomie avec instrumentation postérieure et la laminectomie sur L5, les avantages de la nucléotomie percutanée sont essentiellement les suivants : temps d'intervention court, petites cicatrices, mobilité conservée sans fusion, et absence de fibrose.

Outre les avantages déjà indiqués ci-dessus de l'abord antérieur par rapport à l'abord postéro-latéral du disque, l'abord antérieur présente les suivants :
- absence de gêne par la crête iliaque dans l'approche,
- meilleure décompression des racines nerveuses car le chirurgien a accès aux fragments du disque les plus gênants.

L'invention sera maintenant décrite en référence aux dessins annexés qui en illustrent deux formes de réalisation à titre d'exemples non limitatifs.
La figure 1 est une vue en élévation longitudinale d'une première forme de réalisation de l'instrument de traitement chirurgical selon l'invention, à échelle réduite.
La figure 2 est une vue en élévation suivant la flèche K de la poignée de commande manuelle de l'instrument de la Fig.1.
La figure 3 est une vue en élévation longitudinale partielle à échelle agrandie, de l'extrémité apicale de l'instrument des Fig.1 et 2, équipée d'une forme de réalisation du moyen d'écartement des corps vertébraux.
La figure 4 est une vue de dessus partielle de l'extrémité apicale du tube extérieur représenté à la Fig.3.
La figure 5 est une vue en élévation schématique illustrant le principe de l'abord du disque L5-S1 au moyen de l'instrument des Fig.1 à 4 et sous contrôle laparoscopique.
Les figures 6 et 7 sont des vues en élévation schématique du disque L5-S1, des corps vertébraux voisins et des deux positions possibles du moyen d'écartement de ces corps vertébraux par rotation du tube de travail sur 90° environ.
La figure 8 est une vue en perspective avec arrachement, à échelle réduite, d'une seconde forme de réalisation du tube extérieur de travail de l'instrument selon l'invention, dans laquelle ce tube est double et muni d'un dispositif escamotable pour l'écartement des vertèbres.
La figure 9 est une vue en perspective du tube extérieur de la Fig 8.
La figure 10 est une vue en perspective avec arrachement du tube intérieur de la Fig 8.

La coelioscopie est la technique qui permet le contrôle de l'avancée et le positionnement du dispositif de nucléotomie constitué par l'instrument selon l'invention. Cette technique est habituelle en chirurgie coelioscopique viscérale, étant fréquemment mise en oeuvre pour le petit bassin et le promontoire. Jusqu'à présent elle n'est pratiquement pas connue des chirurgiens orthopédistes, cette technique pouvant du reste ouvrir pour ces derniers d'autres possibilités telles que des arthrodèses.

On rappelle ci-dessous pour mémoire les phases successives de la technique coelioscopique :
- Réalisation d'un pneumo-péritoine par insufflation de gaz carbonique au moyen d'un trocart ombilical.
- Introduction du matériel optique sur la ligne médiane sous-ombilicale.
- Mise en place par une incision de la fosse iliaque gauche, en dehors des vaisseaux épigastriques, d'un trocart permettant l'introduction du matériel chirurgical (pince à disséquer, ciseaux etc).
- Eventuellement par une incision symétrique du flanc droit, mise en place d'un aspirateur qui permet également de refouler les viscères.
- Mise en place d'un trocart sus-pubien médian avec l'aide d'un instrument pour perforer le péritoine. Le matériel optique est alors transféré dans ce trocart, et ainsi situé en face du promontoire.

Le repérage du disque L5-S1 se fait comme en chirurgie "ouverte" en refoulant l'épiploon, le colon transverse et les anses grêles vers le haut. Le promontoire est alors facilement reconnu visuellement, du fait de sa couleur blanche, et tactilement par la consistance élastique particulière du disque intervertébral.

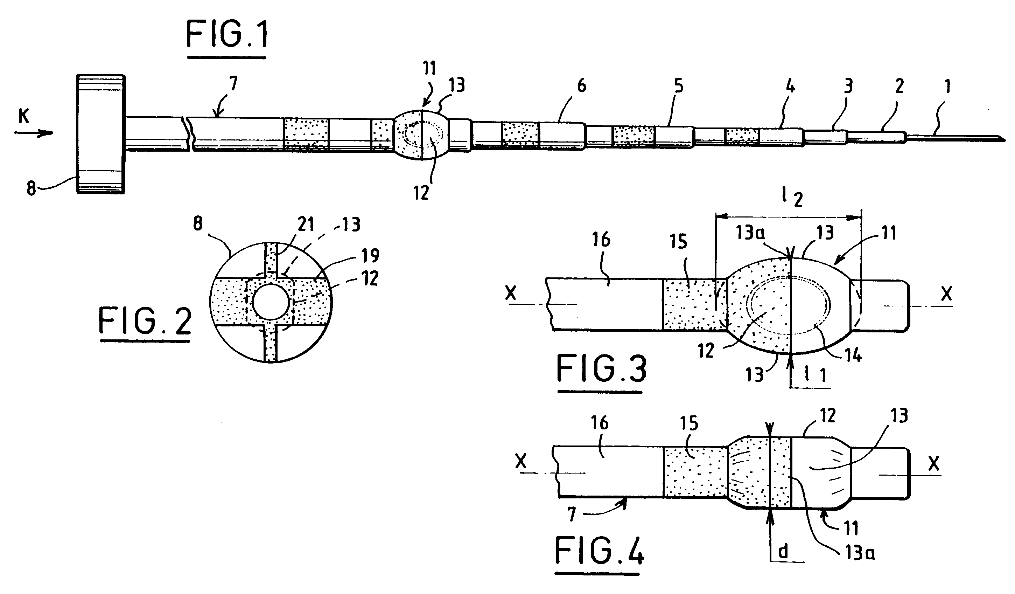

On décrira maintenant l'instrument de traitement chirurgical pour la nucléotomie percutanée, représenté aux Fig.1 à 7.

Cet instrument comprend une aiguille-guide 1, au moins deux tubes gigognes concentriques rectilignes et, dans l'exemple représenté, cinq tubes gigognes 2, 3, 4, 5, 6, de diamètre croissant à partir du tube 2 jusqu'au tube 6, adaptés pour pouvoir être enfilés sur l'aiguille-guide 1 ou trocart, et enfin un tube extérieur de travail 7. Ce dernier est également concentrique au trocart 1 et aux tubes précités 2-6, afin de pouvoir coulisser sur le dernier tube 6.

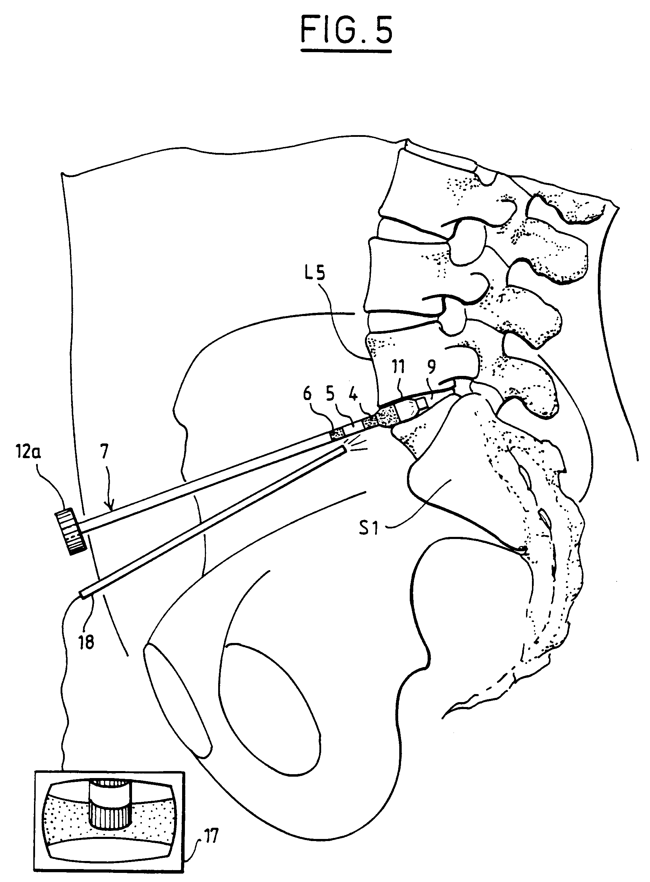

L'extrémité proximale du tube de travail 7 est équipée d'une poignée de préhension manuelle 8, dont la surface est avantageusement moletée. Son extrémité apicale, destinée à pénétrer dans le disque à traiter, par exemple le disque 9 situé entre L5 et S1 (Fig.5 à 7), est pourvue d'un moyen d'écartement des corps vertébraux contigus à ce disque, et de maintien de cet écartement pendant le traitement chirurgical. Dans le mode de réalisation représenté, ce moyen d'écartement comprend une olivette 11 faisant saillie de l'extrémité apicale du tube de travail 7, auquel elle est fixée coaxialement, en étant soit rapportée à celui-ci par tout moyen approprié, soit venue de matière avec ce tube. L'olivette 11 peut également être réalisée dans le même matériau que le tube de travail 7, ou dans un matériau différent. Cette olivette 11 présente deux plats latéraux 12 symétriques par rapport à l'axe XX du tube 7 et de l'instrument dans son ensemble, reliés à deux bossages arrondis 13 symétriques l'un de l'autre par rapport à l'axe XX, et ainsi interposés entre les plats 12. Les plats 12 sont raccordés aux bossages 13 par des bords arrondis 14. La distance d entre les plats 12 est inférieure à la distance l₁ entre les sommets 13a des bossages 13. Ces derniers ont un contour sensiblement ovale, avec un petit axe l₁ et un grand axe l₂, le rapport l₂/l₁ pouvant être avantageusement, mais non limitativement, égal à 2.

Les cinq tubes gigognes 2-6 ont leurs extrémités apicales effilées, mais non tranchantes, et sont avantageusement gradués, de manière connue en soi, ainsi que le tube de travail 7 pourvu de graduations 15, 16. Il en est de même pour le trocart 1, toutes ces graduations permettant au chirurgien de contrôler la progression dans le disque des tubes successifs de l'instrument, grâce à un écran de télévision 17 relié à une sonde coelioscopique 18 introduite dans la cavité péritonéale (Fig.5).

Le tube de travail 7 a une longueur nettement inférieure à celle des autres tubes 2-6 et de l'aiguilleguide 1.

Le trocart 1 doit avoir une flexibilité minimale dans le sens longitudinal et le sens transversal, afin de ne pas risquer de se rompre si la pointe vient au contact d'un corps vertébral, par exemple L5 ou S1. Les tubes 1-6 sont de préférence métalliques, du fait qu'ils doivent avoir leur extrémité apicale effilée pour faciliter leur progression dans les chairs et les parois anatomiques.

L'intervention sur le disque inter-vertébral, par exemple le disque 9 situé en position L5-S1, s'effectue de la manière suivante :
1) on introduit par voie antérieure le trocart 1 jusqu'à ce que son extrémité pénètre à l'intérieur du disque 9 (Fig.5). Sa progression peut être contrôlée par le chirurgien sur l'écran de télévision 7. Le trocart 1 est destiné à servir de guide aux tubes gigognes suivants 2 à 6. On enfile d'abord le premier tube 2 sur le trocart guide 1, puis le tube 3 sur le tube 2, et ainsi de suite jusqu'au tube 6. Les tubes 2 à 6 s'emboîtent exactement les uns dans les autres et pénètrent dans l'annulus en refoulant le tissu péritonéal, sans sectionner aucun élément vasculo-nerveux. Du fait de leur graduation, le chirurgien peut suivre directement sur l'écran de contrôle 17 la pénétration des tubes dans le disque 9, sans qu'il soit nécessaire d'effectuer un contrôle radiographique. Cette pénétration doit être la même que celle du trocart-guide 1.
2) Le tube de travail 7 est ensuite enfilé sur le tube 6. Il est plus court que les tubes précédents 2 à 6, afin de permettre de retirer plus aisément ces derniers du tube de travail 7. En effet une fois le tube 7 mis en place, les autres tubes font saillie de son extrémité proximale, au-delà de la poignée de commande 8.
3) Le chirurgien introduit l'extrémité apicale du tube de travail 7 dans le disque 9 dans sa position angulaire où les deux plats 12 sont à peu près parallèles au plan général du disque 9, donc dans la position angulaire du tube 7 correspondant au moindre écartement entre les corps vertébraux L5 et S1. Cette position angulaire peut être contrôlée par un repère visuel correspondant 19 ménagé diamétralement sur la poignée (Fig. 2) Lorsque le repère 19 s'étend dans une position où il est sensiblement dans le plan général du disque intervertébral 9, il en est de même des plats 12, lesquels ainsi que les bords arrondis 14 facilitent la progression et le positionnement correct de l'extrémité apicale du tube 7 entre les vertèbres. Les bossages 13 s'étendent donc alors sensiblement dans le plan du disque.
4) Il devient maintenant possible au chirurgien de procéder à l'écartement des corps vertébraux, L5 et S1 par exemple. Pour ce faire, le chirurgien fait tourner de 90° la poignée de commande 8, et par conséquent le tube 7 et l'olivette 11. Cette rotation est facilitée par les bords arrondis 14, et les deux bossages ou cames 13 viennent se placer dans un plan sensiblement perpendiculaire au plan général du disque 9. Un second repère 21 ménagé diamétralement sur la poignée 8, perpendiculairement au repère 19 permet au chirurgien de contrôler la position de l'olivette 11 entre les corps vertébraux L5 et S1 : ce repère 21 est pratiquement perpendiculaire au plan général du disque 9 lorsque les bossages 13 sont eux-mêmes perpendiculaires à ce plan. La distance entre les corps vertébraux L5 et S1 augmente alors de d à l_{1,} et ceux-ci restent maintenus dans cette position grâce à la géométrie appropriée de l'olivette 11. Cette dernière permet ainsi d'éviter toute expulsion intempestive du tube provoquée par l'hyperpression intersomatique, qui s'opposerait à une nucléotomie de qualité.

Le trocart-guide 1 et les tubes 2 à 6 étant retirés comme indiqué ci-dessus, le chirurgien peut évacuer le contenu discal par l'intérieur du tube de travail 7, par tout moyen approprié tel que pince disque ou curette.

Il convient de préciser que l'écartement des corps vertébraux doit être réalisé de façon suffisamment contrôlée. La vitesse d'écartement dépend bien entendu de la rapidité de rotation du tube de travail 7, mais aussi du rapport entre le petit axe l₁ et le grand axe l₂ de la section ovale de la came-olivette 11.

La mise en place des instruments qui paraissent nécessaires au chirurgien (arthroscope, laser, nucléotome etc) peut être réalisée en toute sécurité au travers du tube de travail 7. Cet abord permet d'accéder facilement, sans instruments spéciaux, au contenu herniaire.

L'intervention se termine par un rinçage abondant au travers du tube 7, qui est ensuite retiré, et l'hémostase vérifiée. Les instruments coelioscopiques 18, 17 sont également retirés.

Il a été constaté sur un certain nombre de patients que la douleur sciatique dont souffraient ceux-ci a totalement disparu à la suite de ces interventions. Au point de vue post-opératoire, le lever des patients a été autorisé 24 heures après l'opération, et leur retour au domicile s'est fait au troisième jour post-opératoire. Bien entendu, ces patients n'ont été contraints d'utiliser aucun moyen de contention externe ni de pratiquer une rééducation rachidienne.

L'abord antérieur des disques vertébraux et particulièrement des disques L5-S1 sous coelioscopie est donc de réalisation relativement aisée, pour les chirurgiens formés aux techniques coelioscopiques. Il permet de résoudre certains des problèmes rencontrés lors de la réalisation des nucléotomies percutanées L5-S1. L'utilisation d'un matériel adapté augmente la sécurité de cet abord et permet d'envisager une extension de ses indications.

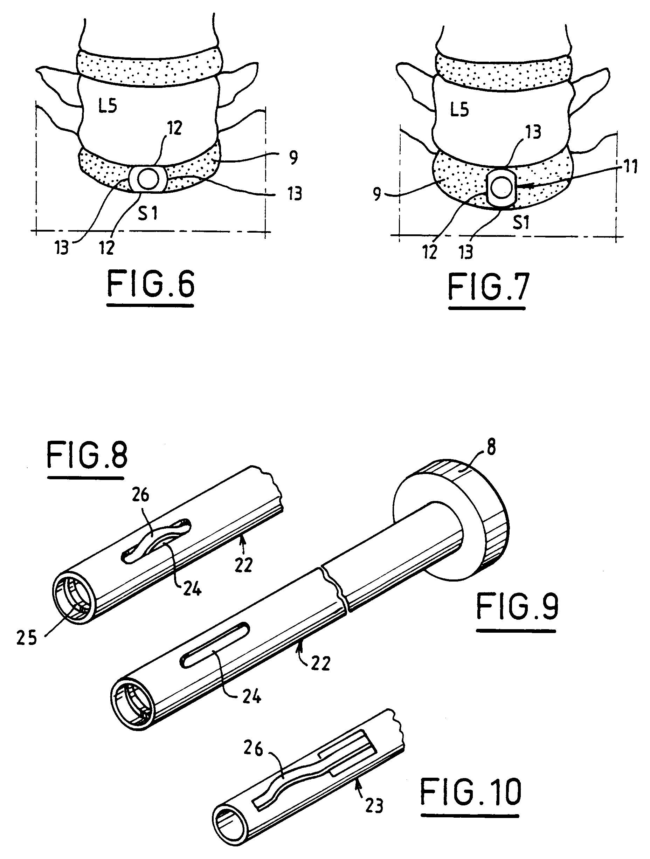

Dans la seconde forme de réalisation de l'instrument selon l'invention, représentée aux Fig.8 à 10, le tube extérieur 7 est remplacé par un tube double constitué de deux éléments tubulaires concentriques 22 et 23 pouvant coulisser axialement l'un par rapport à l'autre. Le tube extérieur 22 est pourvu à son extrémité proximale d'une poignée de commande 8, tandis qu'au moins une lumière 24, de préférence oblongue et s'étendant dans le sens longitudinal du tube, est formée à son extrémité apicale. Entre la lumière 24 et l'extrémité libre du tube 22 est ménagé un épaulement annulaire intérieur 25, faisant saillie dans le tube 22.

L'extrémité apicale de l'élément tubulaire intérieur 23 est pourvue d'au moins une lame 26 élastiquement flexible, s'étendant longitudinalement, et qui fait saillie extérieurement du tube 23 dans sa position détendue. De préférence deux lames ressorts 26 sont ainsi positionnées de manière diamétralement opposée sur l'élément tubulaire 23, afin de coopérer avec deux lumières arrondies 24 de l'élément 22. La ou les lames ressorts 26 sont dimensionnées pour pouvoir traverser la ou les lumières correspondantes 24 et faire saillie latéralement au tube 22, avec une force élastique suffisante lorsque l'élément tubulaire 23 est engagé à coulisse à l'intérieur de l'élément tubulaire 22 jusqu'à ce que les lames ressorts 26, d'abord escamotées, parviennent en regard des lumières 24 (position de la Fig.8) et se détendent élastiquement à travers celles-ci. Dans cette position, l'extrémité libre de l'élément tubulaire 23 vient en butée contre l'épaulement 25, qui bloque l'élément tubulaire 23 et la ou les lames ressorts 26 dans cette position.

L'ensemble des deux éléments tubulaires 22 et 23 avec les lumières 24 et les lames ressorts 26 constitue un dispositif escamotable d'écartement et de maintien des vertèbres. Après introduction du trocart-guide 1 et des tubes gigognes 2 à 6, le chirurgien enfile le tube extérieur 22 sur le tube 6 avec les lumières oblongues 24 positionnées en regard des corps vertébraux L5 et S1. Ensuite il retire les tubes 2 à 6, et introduit dans le tube 22 le tube intérieur 23 jusqu'à ce que les lames 26, escamotées à l'intérieur du 22, se détendent élastiquement à travers les lumières 24, au-delà desquelles elles restent en saillie en provoquant un écartement des corps vertébraux voisins. Les corps vertébraux étant ainsi écartés, la suite de l'intervention se déroule de la même manière que précédemment décrit.

L'avantage du mode de réalisation des Fig.8 à 10 réside notamment dans l'uniformité totale du diamètre du tube extérieur 22 lors de sa progression dans le corps du patient, et donc dans la suppression de zones de plus forte résistance sur la face extérieure du tube. On notera que le moyen de blocage 25 (qui peut être remplacé par tout moyen équivalent) permet de maintenir en toute sécurité la position en saillie des lames flexibles 26.

L'invention est susceptible de diverses variantes d'exécution. Ainsi notamment le nombre des tubes gigognes (2-6) peut varier, et les dispositifs d'écartement des corps vertébraux décrits peuvent être remplacés par tout moyen équivalent. L'olivette 11 peut notamment être remplacée par un seul bossage d'épaisseur double de celle des bossages 13 et la poignée 8 peut être remplacée par un T.

Dans ses différents modes de réalisation possibles, l'invention présente l'avantage de permettre la mise en oeuvre d'un tube extérieur de travail de diamètre nettement plus élevé que celui des tubes de la voie postéro-latérale utilisés jusqu'à présent, qui ne dépassait pas 5mm. De ce fait il devient possible d'introduire dans ce tube extérieur un système motorisé d'extraction par aspiration, d'un diamètre par exemple de 5mm, ce qui n'était pas possible jusqu'à présent. On peut donc utiliser un tube extérieur dont le diamètre peut atteindre la hauteur du disque.

## Revendications

1. Instrument de traitement chirurgical d'un disque intervertébral lombaire par voie antérieure, comprenant une poignée de préhension manuelle et un tube, caractérisé en ce que cet instrument comprend une aiguille-guide (1), au moins deux tubes gigognes concentriques rectilignes (2-6) adaptés pour pouvoir être enfilés sur l'aiguille-guide et un tube extérieur (7) de travail, concentrique aux tubes précités et dans lequel ces derniers peuvent coulisser, ce tube extérieur étant pourvu à son extrémité apicale, destinée à pénétrer dans le disque à traiter, d'un moyen (11; 26, 24) d'écartement des corps vertébraux contigus à ce disque et de maintien de cet écartement (l₁) pendant le traitement chirurgical, tandis que son extrémité proximale est équipée d'une poignée (8) de préhension manuelle.

2. Instrument selon la revendication 1, caractérisé en ce que le moyen d'écartement et de maintien des corps vertébraux écartés comprend une olivette (11) faisant saillie du tube extérieur (7) et fixée coaxialement à ce dernier, et cette olivette présente deux plats latéraux symétriques (12) reliés à deux bossages arrondis symétriques (13), la distance (d) entre les deux plats étant inférieure à la distance (l₁) entre les sommets (13a) des bossages, afin que par rotation du tube extérieur sur 90 degrés à partir de sa position dans laquelle les plats sont parallèles au plan général du disque (9) à traiter, les corps vertébraux sous et sus-jacents puissent être écartés et maintenus en position écartée.

3. Instrument selon la revendication 2, caractérisé en ce que les plats (12) de l'olivette (11) sont raccordés aux bossages (13) par des bords arrondis (14).

4. Instrument selon la revendication 2 caractérisé en ce que les bossages (13) ont un profil sensiblement ovale, avec un rapport déterminé de leur grand axe (l₂) à leur petit axe (l₁).

5. Instrument selon la revendication 4, caractérisé en ce que ledit rapport est égal à 2.

6. Instrument selon la revendication 1, caractérisé en ce que le tube extérieur est double et constitué de deux éléments tubulaires (22, 23) concentriques pouvant coulisser axialement l'un par rapport à l'autre, la poignée de préhension (8) est prévue sur l'extrémité proximale de l'élément tubulaire extérieur (22), dans l'extrémité apicale duquel est ménagée au moins une lumière (24), et l'extrémité apicale de l'élément intérieur (23) est pourvue d'au moins une lame (26) flexible élastiquement, faisant saillie extérieurement audit élément à l'état libre, escamotée lorsque l'élément intérieur (23) est introduit dans l'élément extérieur (22) et pouvant faire saillie de ce dernier à travers la lumière correspondante afin de constituer ledit moyen d' écartement des corps vertébraux lorsque l'élément extérieur est dans la position angulaire voulue entre les vertèbres.

7. Instrument selon la revendication 6, caractérisé en ce qu'il est muni de moyens de butée (25) de l'élément tubulaire intérieur (23) dans l'élément extérieur (22) lorsque la lame flexible (26) est en saillie à travers la lumière associée (24),

8. Instrument selon la revendication 6, caractérisé en ce que le tube extérieur (7) et le tube de travail (22, 23) ont une longueur inférieure à celle des tubes intérieurs (1-6) afin de faciliter l'extraction de ceux-ci du tube extérieur ou du tube de travail.

9. Instrument selon la revendication 8, caractérisé en ce que des repères (19, 21) permettant de contrôler les positions angulaires du moyen (11; 26) d'écartement des corps vertébraux sont agencés sur la poignée (8) de préhension, ainsi que des graduations (15, 16) sur les tubes gigognes (2-6) pour le contrôle coelioscopique de leurs positions.

10. Instrument selon la revendication 1, caractérisé en ce que le moyen d'écartement et de maintien des corps vertébraux écartés comprend un bossage d'épaisseur appropriée à l'écartement souhaité.

11. Instrument selon l'une quelconque des revendications 1 à 9, caractérisé en ce que le tube extérieur (7) a un diamètre pouvant atteindre la hauteur du disque (9) à traiter.

12. Instrument selon la revendication 7, caractérisé en ce que lesdits moyens de butée sont un épaulement annulaire (25) intérieur du tube extérieur (22), ménagé entre la lumière et son extrémité libre.

## Claims

1. Instrument for anterior intervertebral lumbar disc surgery, comprising a manual grip handle and a tube, characterized in that this instrument comprises a guide needle (1), at least two rectilinear concentric extendable tubes (2-6) designed to be able to be engaged on the guide needle, and an outer working tube (7) which is concentric to the aforementioned tubes and in which these tubes can slide, this outer tube being provided at its apical end, intended to penetrate the disc to be treated, with a means (11; 26, 24) for distancing the vertebral bodies contiguous to this disc and for maintaining this distance (l₁) during surgery, while its proximal end is equipped with a manual grip handle (8).

2. Instrument according to Claim 1, characterized in that the means for distancing the vertebral bodies and for maintaining this distance comprises an olive (11) protruding from the outer tube (7) and fixed coaxially to the latter, and this olive has two symmetrical flat side faces (12) connected to two symmetrical rounded bosses (13), the distance (d) between the two flat faces being smaller than the distance (l₁) between the summits (13a) of the bosses. so that, by rotating the outer tube through 90 degrees from its position in which the flat faces are parallel to the general plane of the disc (9) to be treated, the vertebral bodies lying above and below can be distanced and maintained in the distanced position.

3. Instrument according to Claim 2, characterized in that the flat faces (12) of the olive (11) are joined to the bosses (13) via rounded edges (14).

4. Instrument according to Claim 2, characterized in that the bosses (13) have a substantially oval profile, with a defined ratio of their main axis (l₂) to their small axis (l₁).

5. Instrument according to Claim 4, characterized in that the said ratio is equal to 2.

6. Instrument according to Claim 1, characterized in that the outer tube is double and consists of two concentric tubular elements (22, 23) which can slide axially in relation to one another, the grip handle (8) is provided on the proximal end of the outer tubular element (22), in the apical end of which there is formed at least one slot (24), and the apical end of the inner element (23) is provided with at least one elastically flexible blade (26), protruding outside the said element in the free state, retracted when the inner element (23) is introduced into the outer element (22) and being able to protrude from the latter through the corresponding slot in order to constitute the said means for distancing the vertebral bodies when the outer element is in the desired angular position between the vertebrae.

7. Instrument according to Claim 6, characterized in that it is provided with means of abutment (25) of the inner tubular element (23) in the outer element (22) when the flexible blade (26) is protruding through the associated slot (24).

8. Instrument according to Claim 6, characterized in that the outer tube (7) and the working tube (22, 23) have a length which is less than that of the inner tubes (1-6), so as to facilitate the withdrawal of these from the outer tube or the working tube.

9. Instrument according to Claim 8, characterized in that markers (19, 21) permitting monitoring of the angular positions of the means (11; 26) for distancing the vertebral bodies are arranged on the grip handle (8), as well as graduations (15, 16) on the extendable tubes (2-6) for coelioscopic monitoring of their positions.

10. Instrument according to Claim 1, characterized in that the means for distancing the vertebral bodies and for maintaining this distance comprises a boss having a thickness appropriate to the desired distancing.

11. Instrument according to any one of Claims 1 to 9, characterized in that the outer tube (7) has a diameter which can be up to as much as the height of the disc (9) to be treated.

12. Instrument according to Claim 7, characterized in that the said means of abutment are an annular shoulder (25) inside the outer tube (22), formed between the slot and its free end.

## Patentansprüche

1. Gerät zur äußeren chirurgischen Behandlung einer Lendenwirbelscheibe, bestehend aus einem Handgriff und einem Rohr, dadurch gekennzeichnet, daß dieses Gerät eine Führungsnadel (1), mindestens zwei konzentrische, geradlinige, ineinandergeschobene Rohre (2-6), die derart ausgeführt sind, daß sie auf die Führungsnadel aufgeschoben werden können, und ein äußeres Arbeitsrohr (7) umfaßt, das zu den vorgenannten Rohren konzentrisch ist und in dem diese letztgenannten gleiten können, wobei dieses äußere Rohr an seinem vorderen Ende, das dazu bestimmt ist, in die zu behandelnde Scheibe einzudringen, mit einem Mittel (11; 26, 24) zur Entfernung der an dieser Scheibe anliegenden Wirbelkörper und zum Halten dieser Entfernung (l₁) während der chirurgischen Behandlung versehen ist, während sein hinteres Ende mit einem Handgriff (8) versehen ist.

2. Gerät nach Anspruch 1, dadurch gekennzeichnet, daß das Mittel zur Entfernung und zum Halten der voneinander entfernten Wirbelkörper ferner einen ovalen Teil (11) umfaßt, der aus dem äußeren Rohr (7) vorspringt und koaxial zu letztgenanntem befestigt ist, und daß dieser ovale Teil zwei seitliche symmetrische Flächen (12) aufweist, die mit zwei symmetrischen abgerundeten Buckeln (13) verbunden sind, wobei der Abstand (d) zwischen den beiden Flächen kleiner als der Abstand (l₁) zwischen den Spitzen (13a) der Buckel ist, damit durch Drehung des äußeren Rohres um 90 Grad ausgehend von seiner Position, in der die Flächen parallel zu der allgemeinen Ebene der zu behandelnden Scheibe (9) sind, die oben und unten angrenzenden Wirbelkörper voneinander entfernt und in der entfernten Position gehalten werden können.

3. Gerät nach Anspruch 2, dadurch gekennzeichnet, daß die Flachen (12) des ovalen Teils (11) mit den Buckeln (13) durch abgerundete Ränder (14) verbunden sind.

4. Gerät nach Anspruch 2, dadurch gekennzeichnet, daß die Buckel (13) ein im wesentlichen ovales Profil aufweisen, mit einem bestimmten Verhältnis ihrer großen Achse (l₂) zu ihrer kleinen Achse (l₁).

5. Gerät nach Anspruch 4, dadurch gekennzeichnet, daß dieses Verhältnis gleich 2 ist.

6. Gerät nach Anspruch 1, dadurch gekennzeichnet, daß das äußere Rohr doppelt ist und von zwei konzentrischen röhrenförmigen Elementen (22, 23) gebildet wird, die axial zueinander gleiten können, daß der Haltegriff (8) an dem hinteren Ende des äußeren röhrenförmigen Elements (22) vorgesehen ist, in dessen vorderem Ende mindestens eine Öffnung (24) vorgesehen ist, und daß das vordere Ende des inneren Elements (23) mit mindestens einem elastisch biegsamen Plättchen (26) versehen ist, das außen aus dem Element im freien Zustand vorspringt und eingezogen wird, wenn das innere Element (23) in das äußere Element (22) eingeführt wird, und das aus letztgenanntem über die entsprechende Öffnung vorspringen kann, um das Abstandemittel für die Wirbelkörper zu bilden, wenn sich das äußere Element in der gewünschten Winkelposition zwischen den Wirbeln befindet.

7. Gerät nach Anspruch 6, dadurch gekennzeichnet, daß es mit Anschlagmitteln (25) für das innere röhrenförmige Element (23) in dem äußeren Element (22) versehen ist, wenn das biegsame Plättchen (26) über die zugehörige Öffnung (24) vorspringt.

8. Gerät nach Anspruch 6, dadurch gekennzeichnet, daß das äußere Rohr (7) und das Arbeitsrohr (22, 23) eine Länge aufweisen, die geringer als jene der inneren Rohre (1-6) ist, um das Herausziehen derselben aus dem äußeren Rohr oder dem Arbeitsrohr zu erleichtern.

9. Gerät nach Anspruch 8, dadurch gekennzeichnet, daß die Markierungen (19, 21), die es ermöglichen, die Winkelpositionen des Mittels (11; 26) zur Entfernung der Wirbelkörper zu kontrollieren, auf dem Handgriff (8) vorgesehen sind, wie auch Gradeinteilungen (15, 16) auf den ineinandergeschobenen Rohren (2-6) für die zölioskopische Kontrolle ihrer Positionen.

10. Gerät nach Anspruch 1, dadurch gekennzeichnet, daß das Mittel zur Entfernung und zum Halten der voneinander entfernten Wirbelkörper einen Buckel mit einer an die gewünschte Entfernung angepaßten Dicke umfaßt.

11. Gerät nach einem der Ansprüche 1 bis 9, dadurch gekennzeichnet, daß das äußere Rohr (7) einen Durchmesser aufweist, der die Höhe der zu behandelnden Scheibe (9) erreichen kann.

12. Gerät nach Anspruch 7, dadurch gekennzeichnet, daß die Anschlagmittel in einem ringförmigen inneren Ansatz (25) des äußeren Rohres (22) bestehen, der zwischen der Öffnung und seinem freien Ende ausgespart ist.
